# EUROPEAN PATENT SPECIFICATION

(11) **EP 1 658 062 B1**
(45) Date of publication and mention of the grant of the patent: **17.02.2010**
(21) Application number: 04728870.9
(22) Date of filing: 22.04.2004
(51) Int. Cl.: A61K 31/165, A61K 31/381, A61P 1/04, A61P 13/10, A61P 19/02

(54) **ALPHA-AMINOAMIDE DERIVATIVES USEFUL AS ANTI-INFLAMMATORY AGENTS**
ALPHA-AMINOAMID DERIVATE ZUR VERWENDUNG ALS ANTI-INFLAMMATORISCHE WIRKSTOFFE
DERIVES D'ALPHA-AMINOAMIDE UTILISABLES COMME AGENTS ANTI-INFLAMMATOIRES

(30) Priority: 25.08.2003 US 497722 P
(43) Date of publication of application: 24.05.2006
(73) Proprietor: Newron Pharmaceuticals S.p.A., 20091 Bresso (IT)
(72) Inventor: SALVATI, Patricia, I-20020 Arese (IT); VENERONI, Orietta, I-20019 Settimo Milanese (IT); BARBANTI, Elena, I-20093 Cologno Monzese (IT); RUGGERO, Fariello, I-21016 Luino (IT); BENATTI, Luca, I-20093 S. Maurizio al Lambro (IT)
(74) Representative: Minoja, Fabrizio
(86) International application number: PCT/IB2004/001574
(87) International publication number: WO 2005/018627

(56) References cited:
- WO-A-01/45684
- WO-A-99/32462
- WO-A-99/55322
- WO-A-03/020273
- WO-A-03/057147
- WO-A-2004/066987
- WO-A-2004/066990
- VENERONI O ET AL: "Anti-allodynic effect of NW-1029, a novel Na(+) channel blocker, in experimental animal models of inflammatory and neuropathic pain." PAIN. MAR 2003, vol. 102, no. 1-2, March 2003 (2003-03), pages 17-25, XP002300721 ISSN: 0304-3959

## Description

### FIELD OF THE INVENTION:

The invention relates to α-aminoamide derivatives, a chemical class of sodium channel blockers, which are useful as anti-inflammatory agents. Particularly, the invention relates to their use as therapeutic anti-inflammatory agents and to pharmaceutical compositions containing them.

### BACKGROUND OF THE INVENTION

Inflammation produces profound changes in the excitability of primary afferent neurons innervating the inflamed tissue. These changes underlie the initiation and maintenance of chronic inflammatory state. Studies have shown that post-translational modification or abnormal expression of sodium channels in dorsal root ganglion (DRG) neurons occurs after tissue inflammation.

Inflammation and inflammation-induced tissue damage is believed to happen in multiple and diverse ways. In one example, sodium channels are substantially up-regulated in inflamed tissues. Carrageenan injection into the plantar surface of the rat hind paw, used as an animal model of inflammation, induces edema, hyperthermia and hyperalgesia. Although sodium channel blockers may be effective in neuropathic pain relief, not all exert an evident anti-inflammatory action. In fact, two sodium channel blockers, crobenetine and mexeletine, were able to reverse the mechanical hyperalgesia without any effect on swelling and stiffness of the inflamed joint induced by carrageenan. Hence, these findings indicate that the analgesic activity of sodium channel blockers is not necessarily related to an anti-inflammatory property.

At the same time, however, inflammatory mediators such as substance P and calcitonin gene-related peptide (CGRP), which are involved in nociceptive transmission, increase in DRG neurons following inflammation. Substance P plays an important role in the induction of neurogenic inflammation and it has been shown to exert potent pro-inflammatory action such as vasodilatation, increased capillary permeability, and the secretion of prostaglandin E₂.

PCT patent publications WO90/14334, WO94/22808, WO97/05102, WO 97/0511 and WO 99/35215, the text of which are incorporated by reference herein, disclose substituted benzylaminopropionamide compounds active on the central nervous system and useful as anti-epileptic, anti-Parkinson, neuroprotective, antidepressant, and antispastic hypnotic agents (*see also* Povarollo P. et al. (1998), "Synthetic and anticonvulcant activity of a new class of 2-[(arylalkyl)amino]alkanamide derivatives", J. Med. Chemistry, 41: 579-590). WO99/35125 and WO99/35123 disclose substituted benzylaminopropanamide compounds active on the central nervous system and useful as analgesic agents (*see also* Veneroni O. et al, (2003) "Anti-allodynic effect of NW-1029, a novel Na+ channel blocker, in experimental animal models of inflammatory and neuropathic pain", Pain 102(1-2):17-25).

U.S. Patent No. 3,549,690 to Leigh et al. further describes carboxylic acid derivatives of the following general formula: 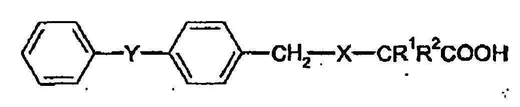 which lower the concentration of cholesterol and/or triglycerides in blood serum and which possess anti-inflammatory activity. U.S. Patent No. 6,548,507 to Bountra et al. relates to the use of sodium channel antagonists for the treatment of diseases mediated by, or exacerbated by, neuronal apoptosis, in particular sensory neuronal apoptosis.

WO 03/020273 discloses the use of alpha-aminoamides in combination with gabapentin for the treatment of pain conditions. Veneroni O et al., Pain 102 (2003) 17-25, discloses the use of salfinamide for treating neurogenic inflammation induced by the formalin test in the mouse.

WO 03/0571747, WO 99/32462 and WO 99/55322 disclose respectively aroyl pyrrole heteroaryl methanone derivatives, triazine derivatives and remacemide having anti-inflammatory effects and sodium channel blocking activity.

Laird J. M. A. et al., B. J. of Pharmacology (2001) 134, 1742-1748 and Lu Ying et al., J. of Pharmacology and Experimental Therapeutics (1999) 290, 1, 214-219 disclose sodium calcium blockers (nexelitine, crobenetine and gabapentin) having anti-inflammatory effects.

WO 01/45684 discloses anti-inflammatory activity of a combination of sodium channel blockers and adenosine A1 agonists.

WO 2004/066990 and WO 2004/066987 disclose the use of salfinamide for the treatment of urinary tract disorders and of G1 tract disorders.

### SUMMARY OF THE INVENTION

Despite the large number of available anti-inflammatory agents, however, the use of such anti-inflammatory agents is limited by severe side effects and/or modest activity in some inflammation conditions. For example, adverse side effects in the gastrointestinal tract are commonly induced by certain levels of classical anti-inflammatory drugs like indomethacin, a non-steroidal anti-inflammatory drug (NSAID). Similarly, COX-2 inhibitors only partially reduce inflammatory disorders. Thus, there is still a clear need to develop new compounds with better therapeutic index in treating inflammatory disorders. The present invention provides rapid and highly effective methods for treating a variety of inflammatory disorders from body organs and systems by utilizing, *in vivo,* certain α-aminoamide compounds of the invention in a therapy which is a superior alternative to existing treatments.

In an embodiment, the invention includes the use of at least one α-aminoamide compound of formula (I): 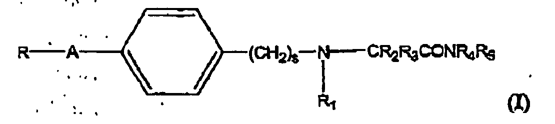 wherein:
■ A is a -(CH₂)ₙ-X- group, wherein n is an integer of 0 to 5, X is CH₂, -O-, -S- or -NH-;
■ s is 1 or 2;
■ R is a furyl, thienyl, or pyridyl ring or a phenyl ring, optionally substituted by one or two substituents independently selected prom halogen, hydroxy, cyano, C₁-C₆ alkyl, C₁-C₆ alkoxy or trifluoromethyl;
■ R₁ is hydrogen or C₁-C₆ alkyl or C₃-C₇ cycloalkyl;
■ R₂ and R₃ are independently selected from hydrogen; C₁-C₄ alkyl, optionally substituted by hydroxy or phenyl; phenol, optionally substituted by one or two substituents independently selected from C₁-C₆ alkyl, halogen, hydroxy, C₁-C₆ alkoxy or trifluoromethyl; or R₂ and R₃, taken with the carbon atom which they are linked to, form a C₃-C₆ cycloalkyl ring; and
■ R₄, R₅ are, independently, hydrogen, C₁-C₆ alkyl or C₃-C₇ cycloalkyl; or R₄ and R₅, taken together with the nitrogen atom they are linked to, form a 5-7 atom saturated heterocyclic ring;
or isomers, mixtures, and pharmaceutically acceptable salts thereof as an inflammatory agent for the preparation of a medicament for the treatment of anti-inflammatory disorders.

The alkyl and alkoxy groups can be branched or can be straight chain groups.

In an embodiment of the invention, when n as 1, s is 1, X is O, R₁, R₂, R₄ and R₅ are H and R₃ is CH₃, R is not an *m*-fluoro-sabstitated phenyl ring.

Pharmaceutically acceptable salts of the compounds of the invention include, for example, acid addition salts with inorganic acids, *e*.*g*., nitirc, hydrochloric, hydrobromic, sulfuric and phosphoric acids and the like, or organic acids, *e*.*g*., acetic, propionio, glycolic, lactic, oxalic, malonic, malic, tartaric, citric, succinic, benzoic, cinnamic, mandelic, methanesulfosic, p-toluenesulfonic and salicylic acids, and the like.

Some of the compounds of formula (I) can have asymmetric carbon atoms, and therefore can exist either as racemic mixtures or as individual optical isomers (enantiomers). Accordingly, the term "pharmaceutically acceptable salts" of the α-aminoamide of formula (I) is also meant to include within its scope all the possible isomers and their mixtures, and any pharmaceutically acceptable metabolite, bioprecursor and/or pro-drug, *i*.*e*., a compound which has a structural formula different from the one of the α-aminoamide of formula (I), and yet is directly or indirectly converted *in vivo* into a compound having formula (I), upon administration to a mammal, particularly a human being.

Preferred compounds of formula (I) include those wherein A is a group chosen from - CH₂-, -CH₂-CH₂-, -CH₂-S-, -CH₂-CH₂-S-, and -(CH₂)ₙ-O-, wherein n is an integer of 1 to 5;
■ s is 1 or 2;
■ R is a phenyl ring, optionally substituted by one or two substituents independently selected from a halogen, trifluoromethyl, a methoxy, or a thienyl ring;
■ R₁ is hydrogen or C₁-C₄ alkyl;
■ one of R₂ and R₃ is hydrogen and the other is C₁-C₄ alkyl, optionally substituted by hydroxy or phenyl, optionally substituted by one or two halogen atoms, or R₂ and R₃ are both methyl, or together they can form with the atom they are linked to a cyclopropyl or a cyclopentyl ring; and
■ R₄, R₅ are hydrogen or C₁-C₄ alkyl, or, together with the nitrogen they are linked to, form a pyrrolidine or piperidine ring, and the pharmaceutically acceptable salts thereof.

Examples of specific compounds of formula (I) - which can be used singly or in combination with other compounds of formula (1) - in an effective amount for treating one or more inflammatory disorders in a patient include, but are not limited to:
2-(4-Benzyloxybenzylamino)-propanamide;
2-[4-(2-Methoxybenzyloxy)-benzylamino]-propanamide;
2-[4-(2-Fluorobenzyloxy)-benzylamino]-propanamide;
(S)-(+)-2-[4-(Z-Fluorobenzyloxy)-benzylamino]-propanamide;
2-[4-(2-Fluorobenzyloxy)-benzylamino]-2-methyl-propanamide;
(S)-(+)-2-[4-(3-Fluorobenzyloxy)-benzylamino]-propanamide, methanesulfonate;
2-[4-(2-Fluorobenzyloxy)-benzylamino]-N-methyl-propanamide;
N-(2-[4-(2-Fluorobenzyloxy)-benzylamino])-propionyl-pyrrolidine;
2-[4-(3-Methoxybenzyloxy)-benzylamino]-propanamide;
2-[4-(3-Cyanobenzyloxy)-benzylamino]-propanamide;
2-[4-(3-Fluorobenzyloxy)-benzylamino]-propanamide;
(S)-(+)-2-[4-(3-Fluorobenzyloxy)-benzylamino]-propanamide;
2-[4-(3-Fluorobenzyloxy)-benzylamino]-2-methyl-propanamide;
2-[4-(3-Fluorobenzyloxy)-benzylamino]-N-methyl-propanamide;
N-{2-[4-(3-Fluorobenzyloxy)-benzylamino]}-propionyl-pyrrolidine;
2-[4-(4-Fluorobenzyloxy)-benzylamino]-propanamide;
2-[4-(3-Fluorobenzyloxy)-benzylamino]-2-methyl-propanamide;
2-[4-(2-Chlorobenzyloxy)-benzylamino]-propanamide;
2-[4-(3-Chlorobenzyloxy)-benzylamino]-propanamide;
2-(4-Benzyloxybenzylamino)-3-hydroxy-propanamide;
2-[4-(2-Fluorobenzyloxy)-benzylamino]-3-hydroxy-propanamide;
2-[4-(3-Fluorobenzyloxy)-benzylamino]-3-hydroxy-propanamide;
2-(4-Benzyloxybenzylamino)-3-bydroxy-N-methyl-propanamide;
2-[4-(2-Fluorobenzyloxy)-benzylamino]-3-hydroxy-N-methyl-propanarnide;
2-[4-(3-Fluoroben2yloxy)-benzylammo]-3-hydroxy-N-methyl-propanamide;
2-[4-(2-Chlorobenzyloxy)-benzylamino]-3-hydroxy-N-methyl-propanamide;
2-[4-(3-Cyanobenzyloxy)-benzylamino]-3-hydroxy-N-methyl-propanamide;
2-[4-(3-Cyanobenzyloxy)-benzylamino]-2-meihyl-3-hydtoxy-N-methyl-propanamide;
2-[4-(3-Chlorobenzyloxy)-phenylethylamino]-propanamide;
2-{4-[2-(3-Fluorophenyl)-ethyloxy]benzylamino}-propanamide;
2-{4-[2-(3-Fluorophenyl)-ethyl]benzylamino}-propanamide;
2-[N-(4-Benzyloxybenzyl)-N-methylamino]-propanamide;
2-{4-[(3-Chlorobenzyloxy)-phenylethyl]-amino}-propanamide;
2-[4-Benzylthiobenzylamino]-propanamide;
2-[4-(2-Fluorobenzylthio)-benzylamino]-propanamide;
2-[4-(3-Fluorobenzylthio)-benzylamino]-propanamide;
2-[4-(3-Phenylpropyloxy)-benzylamino]-propanamide;
2-[4-(4-Phenylbutyloxy)-benzylamino]-propanamide;
2-[4-(5-Phenylpentyloxy)-benzylamino]-propanamide;
2-(4-Benzyloxybenzylamino)-3-phenyl-N-methyl-propanamide;
2-(4-Benzyloxybenzylamino)-3-methyl-N-methyl-butanamide;
2-(4-Benzyloxybenzylamino)-2-phenyl-acetamide;
2-[4-(2-Fluorobenzyloxy)-benzylamino]-2-phenyl-acetamide;
2-[4-(3-Fluorobenzyloxy)-benzylamino]-2-phenyl-acetamide;
2-[4-(2-Fluorobenzyloxy)-benzyl-N-methylamino]-2-phenyl-acetamide;
2-[4-(3-Fluorobenzyloxy)-benzyl-N-methylamino]-2-phenyl-acetamide;
2-[4-(3-Chlorobenzyloxy)-benzylamino]-2-phenyl-acetamide;
2-[4-(2-Fluorobenzyloxy)-benzylamino]-2-(2-fluorophenyl)-acetamide;
2-[4-(2-Fluorobenzyloxy)-benzylamino]-2-(3-fluorophenyl)-acetamide;
2-[4-(3-Fluorobenzyloxy)-benzylamino]-2-(2-fluorophenyl)-acetamide;
2-[4-(3-Fluorobenzyloxy)-benzylamino]-2-(3-fluorophenyl)-acetamide;
2-[4-(3-Chlorobenzyloxy)-benzylamino]-2-(3-fluorophenyl)-acetamide;
2-(4-(2-Thienyloxy)-benzylamino)-propanamide;
or isomers, mixtures, and pharmaceutically acceptable salts thereof.

A preferred compound of formula (I), which can be used singly, or in combination with other compounds of formula (I), in an effective amount for treating one or more inflammatory disorders in a patient is (S)-(+)-2-[4-(2-Fluorobenzyloxy)-benzylamino]-propanamide, or a pharmaceutically acceptable salt thereof.

In one embodiment the patient being treated is a mammal, including humans, in need of alleviation, prevention, or inhibition of symptoms of one or more inflammatory disorders.

Particularly, the mammal in need of the above mentioned treatment is administered a dose of an α-aminoamide of formula (I) as above defined which ranges from about 0.3 to about 100 mg/kg of body weight per day. "Treatment" as used herein includes any care by procedures or applications to a mammal, and particularly a human, that are intended to a) prevent the disease or disorder from occurring in a subject that may be predisposed to the disease/disorder, but has not yet been diagnosed with having it; b) inhibiting the disease/disorder, or condition, *i*.*e*., arresting its development; or c) relieving the disease/disorder, or condition, *i*.*e*., causing regression of the disease/disorder, or condition.

Inflammatory conditions in a mammal, including humans, can thus be inhibited, alleviated and prevented. Examples of inflammation conditions in mammals which can be treated by administering one or more α-aminoamide compounds of formula (I) include, but are not limited to: arthritic conditions such as alkylosing spondylitis, cervical arthritis, fibromyalgia, gut, juvenile rheumatoid arthritis, lumbosacral arthritis, osteoarthritis, osteoporosis, psoriatic arthritis, rheumatic disease, rheumatoid arthritis, eczema, psoriasis, dermatitis and inflammatory conditions such as sunburn; inflammatory eye conditions such as uveitis and conjunctivitis; lung disorders in which inflammation is involved such as asthma and bronchitis; conditions of gastro-intestinal tract including ulcers, gingivitis, Crohn's disease, atrophic gastritis, gastritis varialoforme, ulcerative colitis, celiac disease, regional iletis, peptic ulceration, pyresis, and other damage to the GI tract, for example, by *Helicobacter pylori*; visceral inflammation such as bladder irritation and cystitis; inflammatory neurological disorders of the central or peripheral nervous system; multiple sclerosis; inflammatory neuropathies and neurological complication of AIDS, inflammation associated with autoimmune diseases, to trauma including trauma generated by surgery, infections, metabolic disorders, and tumors.

In another aspect, the invention includes an α-aminoamide of formula (I) administered as the active agent of a pharmaceutically acceptable composition having anti-inflammatory activity which can be prepared by conventional procedures known in the art, for instance by mixing the active agent with a pharmaceutically acceptable, therapeutically inert organic and/or inorganic carrier or excipient materials.

### DETAILED DESCRIPTION OF THE INVENTION

A preferred compound of formula (I), used in an effective amount for treating one or more inflammatory disorders in a patient is (S)-(+)-2-[4-(2-fluorobenzyloxy)-benzylamino]-propanamide. The compounds of formula (I), and the pharmaceutically acceptable salts thereof, may be obtained by well known processes as described in the international applications cited above.

Combination therapy" (or "co-therapy") includes the administration of an alpha-aminoamide compound of formula (I) of the invention and at least a second agent as part of a specific treatment regimen intended to provide the beneficial effect from the co-action of these therapeutic agents. Benefits of such combinations include reduction of the dose of conventional inflammatory agents (*i*.*e*., other than the agents of the present invention) with consequent reduction of the side-effects of such conventional agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations contemplated by the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally.

Alternatively, for example, all therapeutic agents may be administered orally or all therapeutic agents may be administered by intravenous injection. The sequence in which the therapeutic agents are administered is not narrowly critical. "Combination therapy" also can embrace the administration of the therapeutic agents as described above in further combination with other biologically active ingredients and non-drug therapies (*e*.*g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The α-aminoamide compositions of the invention can be administered in a variety of dosage forms, *e*.*g*., orally, in the form of tablets, troches, capsules, sugar or film coated tablets, liquid solutions, emulsions or suspensions; rectally, in the form of suppositories; parenterally, *e*.*g*., by intramuscular or intravenous injection or infusion; and transdermally in the form of a patch, ointment, emulsion, lotion, solution, gel, cream and nasal spray.

Suitable pharmaceutically acceptable, therapeutically inert organic and/or inorganic carrier or excipient materials useful in the preparation of such composition include, for example, water, gelatin, gum arabic, lactose, starch, cellulose, magnesium stearate, talc, vegetable oils, polyalkyleneglycols and the like. The α-aminoamide compositions of formula (I) can be sterilized and may contain further components, well known to those skilled in the art, such as, for example, preservatives, stabilizers, wetting or emulsifying agents, *e*.*g*., paraffin oil, mannide monooleate, salts to adjust osmotic pressure, buffers and the like.

Additionally, the solid oral forms can contain, together with the active agent, diluents, *e*.*g*., lactose, dextrose, saccharose, cellulose, corn starch or potato starch; lubricants, *e*.*g*., silica, talc, stearic acid, magnesium or calcium stearate, and/or polyethylene glycols; binding agents, *e*.*g*., starches, arabic gums, gelatin, methylcellulose, carboxymethylcellulose or polyvinyl pyrrolidone; disaggregating agents, *e*.*g*., a starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. The pharmaceutical preparations may be manufactured in any known manner, for example, by means of mixing, granulating, tableting, sugar-coating, or film-coating processes.

The oral formulations comprise sustained release formulations which can be prepared in a conventional manner, for instance by applying an enteric coating to tablets and granules.

The liquid dispersion for oral administration may be *e*.*g*., syrups, emulsions and suspension. The syrups may further contain as a carrier, for example, saccharose or saccharose with glycerine and/or mannitol and/or sorbitol.

Suspensions and emulsions may contain as a carrier, for example, a natural gum, agar, sodium alginate, pectin, methylcellulose, carboxymethyl-cellulose, or polyvinyl alcohol. The suspensions or solutions for intramuscular injections may contain, together with the active compound, a pharmaceutically acceptable carrier, *e*.*g*., sterile water, olive oil, ethyl oleate, glycols, *e*.*g*., propylene glycol, and, if desired, a suitable amount of lidocaine hydrochloride. The solutions for intravenous injections or infusion may contain as a carrier, for example, sterile water or preferably they may be in the form of sterile, aqueous, or isotonic saline solutions.

The suppositories may contain, together with the active agent, a pharmaceutically acceptable carrier, *e.g*., cocoa butter, polyethylene glycol, a polyoxyethylene sorbitan fatty acid ester surfactant or lecithin.

Compositions including α-aminoamides of formula (I) are generally in the form of a dose unit containing, for example, 21 to 7000 mg of active ingredient per unit dosage form. Suitable treatment is given 1 or 2 or 3 times daily, depending upon clearance rate. Accordingly, the desired dose may be presented in a single dose or as divided doses administered at appropriate intervals, for example, two to four or more sub-doses per day.

The pharmaceutical compositions including an α-aminoamide of formula (I) can contain, per dosage unit, *e.g*., capsule, tablet, powder injection, teaspoonful, suppository and the like, from about 21 to 7000 mg of the active agent.

Optimal therapeutically effective doses to be administered may be readily determined by those skilled if the art and will vary, basically, with the strength of the preparation, with the mode of administration and with the advancement of the inflammatory condition or disorder treated. In addition, factors associated with the particular subject being treated, including subject age, weight, diet and time of administation, will result in the need to adjust me dose to an appropriate therapeutically effective level.

The advantages derived from the uses and the methods of the invention as above defined are many, and include the possibility to prevent and treat basically all types of inflammation disorders.

Surprisingly, the use of the α-aminoamides of formula (I) as set forth herein does not show relevant adverse side effects at gastrointestinal levels that are commonly induced by classical anti-inflammatory drugs, *e*.*g*., NSAIDs like indomethacin, and COX-2 inhibitors, that only partially reduce they.

The following EXAMPLES are prevented in order to more fully illustrate the preferred embodiments of the invention.

### EXAMPLES

### EXAMPLE 1. Paw edema catraaeenaa-induced inflammation

The anti-inflammatory activity of the α-aminoamide compounds of formula (I) have proven effective in a rat model of inflammation induced by carrageenan infection. The α-aminoamide compounds disclosed herein have been found to be active in inhibiting the paw edema formation after injection of carrageenan and the *in vitro* substance P (SP) release, and are therefore deemed to be useful as anti-inflammatory agents generally.

The potential anti-inflammatory effect of (S)-(+)-2-[4-(2-fluorobenzyloxy)-benzylamino]-propanamide ("compound A") was investigated in the rat model of inflammatory acute pain induced by subplantar injection of carrageenan. Intraplantar injection of carrageenan elicit a time-dependent increase in paw volume.

### Procedure:

Male Wistar rats of 175-200 grams were used. The left hind paw was injected with 100 pl of carrageenan (2 % w/v in saline). Compound A (30 mglkg), indomethacin (5 mg/kg), or control vehicle (such as distilled water) were orally administered 1 h before carrageenan injection. The paw volume was measured with a plethysmometer (Ugo Basile) immediately before (basal) and 1, 2, 3, 4 and 5h after the carrageenan injection.

### Results:

In the control group carrageenan injection resulted in a time-related increase in ipsilateral hindpaw volume of 1.02 ml at 5 h after carrageenan injection. Compound A (30 mg/kg) prevented paw edema formation at all time points considered. Notably, the inhibition was maximal at 4 h after carrageenan injection with a 40 % reduction of edema vs. the control vehicle. Similarly, indomethacin (5 mg/kg) was able to prevent paw edema formation yielding an inhibition of about 50 % at the same time point. Data are reported in Table 1.

**TABLE 1:**

| Effect of Compound A (30 mg/kg *po*) and indomethacin (5 mg/kg *po*) on volume of paw edema (ml) induced by carrageenan. | | | | | |
|---|---|---|---|---|---|
| | **60'** | **120'** | **180'** | **240'** | **300'** |
| | | | | | |
| Control Vehicle | 0.35 ± 0.03 | 0.54 ± 0.04 | 0.72 ± 0.03 | 0.92 ± 0.03 | 1.02 ± 0.04 |
| | | | | | |
| Compound A 30 mg/Kg | 0.19 ± 0.03 | 0.32*± 0.03 | 0.44*** ± 0.03 | 0.56*** ± 0.04 | 0.70**** ± 0.05 |
| | | | | | |
| Indomethacin 5 mg/Kg | 0.29 ± 0.04 | 0.31*± 0.04 | 0.38*** ± 0.05 | 0.45*** ± 0.05 | 0.57*** ± 0.07 |
| | | | | | |

Data are expressed as mean Δml± s.e. of 13/15 rats and represent the volume difference in paw edema at different time points after carrageenan injection with respect to the basal paw volume measured before treatment. Data are evaluated by two-way analysis of variance followed by Bonferroni test. * p<0.05; ***p<0.001 vs. vehicle

### EXAMPLE 2. Determination of substance P (SP) release from rat spinal cord synaptosomes

### Procedure:

Male adult Sprague-Dawley rats were used. Following decapitation, the spinal cord was removed and homogenized in sucrose buffer 0.32 M, pH 7.4. Samples were centrifuged at 12000 g for 20 minutes and the synaptosomal fraction was resuspended in physiological buffer. SP release from spinal cord superfused synaptosomes was induced by KCI (35 mM) and measured by RIA method (*see* Lee CM. et al. (1980) "The development and application of a novel N-terminal directed substance P antiserum", Life Science 27(7):535-543).

### Results:

*In vitro,* Compound A was very potent in reducing the evoked SP release from spinal cord superfused synaptosomes in a concentratioa-related manner ranging from 0.1 to 30 µM with an IC₅₀ of 2.12 µM. SP is one of those substances referred to as cytokines, that are mediators of inflammation. SP is a prime link in the chain of events that, after the interaction between a noxa with tissues or cells of the host, leads to inflammatory damage and symptoms of inflammation. The ability to inhibit release of SP is an important step in reducing inflammation-related damage and symptoms.

## Claims

1. The use of an alpha-aminoamide compound of formula (I), 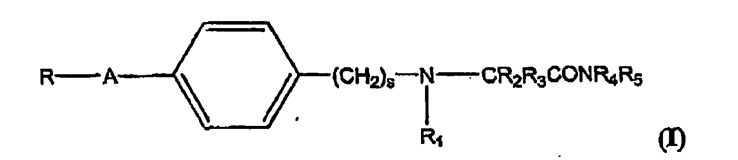 wherein:
■ A is a -(CH₂)ₙ-X- group, wherein n is an integer of 0 to 5, X is CH₂, -O- -S- or -NH-;
■ s is 1 or 2;
■ R is a furyl, thienyl, or pyridyl ring or a phenyl ring, optionally substituted by one or two substituents independently selected from halogen, hydroxy, cyano, C₁-C₆ alkyl, C₁-C₆ alkoxy or trifluoromethyl;
■ R₁ is hydrogen or C₁-C₆ alkyl or C₃-C₇ cycloalkyl;
■ R₂ and R₃ are independently selected from hydrogen; C₁-C₄ alkyl, optionally substituted by hydroxy or phenyl; phenyl, optionally substituted by one or two substituents independently selected from C₁-C₆ alkyl, halogen, hydroxy, C₁-C₆ alkoxy or trifluoromethyl; or R₂ and R₃, taken with the carbon atom which they are linked to, form a C₃-C₆ cycloalkyl ring; and
■ Rid, R₅ are, independently, hydrogen, C₁-C₆ alkyl or C₃-C₇ cycloalkyl; or R₄ and R₅, taken together with the nitrogen atom they are linked to, a 5-7 atom saturated heterocyclic ring;
or isomers, mixtures, and pharmaceutically acceptable salts or esters thereof, **as an anti-inflammatory agent**, for the preparation of a medicament for the treatment of inflammatory disorders.

2. The use of claim 1, wherein A is selected from -CH₂-,-CH₂-CH₂-,-CH₂-S-,-CH₂- CH₂-S-or-(CH₂)ₙ-0-; n is an integer from 0 to 5; s is 1 or 2; R is a phenyl ring, optionally substituted by one or two substituents independently selected from halogen, trifluoromethyl, a methoxy, or a thienyl ring; R₁ is hydrogen or C₁-C₄ alkyl; one of R₂ and R₃ is hydrogen and the other is C₁-C₄ alkyl, optionally substituted by hydroxy or phenyl, or phenyl, optionally substituted by one or two halogen atoms, or R₂ and R₃ are both methyl or together **can form** with the atom they are linked to a cyclopropyl or a cyclopentyl ring; and R₄, R₅ are hydrogen or C₁-C₄ alkyl or together with the nitrogen they are linked to, form a pyrrolidine or piperidine ring.

3. The use of claim 1, wherein said compounds is administered at a dose ranging from about 0.3 to about 100 mg/kg body weight per **day in a mammal.**

4. The use of claim 1, wherein said inflammatory disorders are selected from the group consisting of: ankylosing spondylitis; cervical arthritis; fibromyalgia; gut; juvenile rheumatoid arthritis; lumbosacral arthritis; osteoarthritis; osteoporosis; psoriatic arthritis; rheumatic disease; rheumatoid arthritis; eczema; psoriasis; dermatitis; sunburn; inflammatory eye conditions; uveitis; conjunctivitis; inflammatory lung disorders; asthma; bronchitis; ulcers; gingivitis; Crohn's disease; atrophic gastritis; gastritis varialoforme; ulcerative colitis; celiac disease; regional ileitis; peptic ulceration; pyresis; inflammation of the GI tract due to Helicobacter pylori; visceral inflammation; bladder irritation; cystitis; inflammatory neurological disorders of the central or peripheral nervous system; multiple sclerosis; inflammatory neuropathies; neurological complication of AIDS, and other diseases or disorders associated with inflammation.

5. The use according to any one of claims 1-4 wherein the compound is selected from:
2-(4-Benzyloxybenzylamino)-propanamide;
2-[4- (2-Methoxybenzyloxy)-benzylamino]-propanamide;
2-[4-(2-Fluorobenzyloxy)-benzylaznino]-propanarnide;
(S)-(+)-2-[4-(2-Fluorobenzyloxy)-benzylamino]-propanamide;
2-[4-(2-Fluorobenzyloxy)-benzylamino]-2-methyl-propanamide;
(S)-(+)-2-j4-(3-Fluorobenzyloxy)-benzylamino-propanamide
methanesulfonate; 2-[4-(2-Fluorobenzyloxy)-benzylamino]-N-methyl-propanamide;
N'-{2-[4-(2-Fluorobenzyloxy)-benzylamino]}-propionyl-pyrrolidine;
2-[4-(3-Methoxybenzyloxy)-benzylamino]-propanamide;
2-[4-(3-Cyanobenzyloxy)-benzylamino]-propanamide;
2-[4-(3-Fluorobenzyloxy)-benzylamino]-propanamide;
2-[4-(3-Fluorobenzyloxy)-benzylamino]-2-methyl-propanamide;
2-[4-(3-Fluerobeazyloxy)-benzylamino]-N-methyl-propanamide;
N-{2-[4-(3-Fluorobenzyloxy)-benzylamino]}-propionyl-pyrrolidine;
2-[4-(4-Fluorobenzyloxy)-benzylamino]-propanamide;
2-[4-(3-Fluorobenzyloxy)-benzylamino]-2-methyl-propanamide;
2-[4-(2-Chlorobenzyloxy)-benzylamino]-propanamide;
2-[4-(3-Chlorobenzyloxy)-benzylamino]-propanamide;
2-(4-Benzyloxybenzylamino)-3-hydroxy-propanamide;
2-[4-(2-Fluorobenzyloxy)-benzylamino]-3-hydroxy-propanamide;
2-[4-(3-Fluorobenzyloxy)-benzylamino]-3-hydroxy-propanamide;
2-(4-Benzyloxybenzylamino)-3-hydroxy-N-methyl-propanamide;
2-[4-(2-Fluorobenzyloxy)-benzylamino]-3-hydroxy-N-methyl-propanamide;
2-[4-(3-Fluorobenzyloxy)-benzylamino]-3-hydroxy-N-methyl-propanamide;
2-[4-(2-Chlorobenzyloxy)-benzylamino]-3-hydroxy-N-methyl-propanamide;
2-[4-(3-Cyanobenzyloxy)-benzylamino]-3-hydroxy-N-methyl-propanamide;
2-[4-(3-Cyanobenzyloxy)-benzylamino]-2-methyl-3-hydroxy-N-methyl-propanamide;
2-[4-(3-Chlorobenzyloxy)-phenylethylamino]-propanamide;
2-{4-[2-(3-Fluorophenyl)-ethyloxy]benzylamino}-propanamide;
2-{4-[2-(3-Fluorophenyl)-ethyl]benzylamino}-propaamide;
2-[N-(4-Benzyloxybenzyl)-N-methylamino]-propanamide;
2-{4-[(3-Chlorobenzyloxy)-phenylethyl]-amino}-propanamide;
2-[4-Benzylthiobenzylamino]-propanamide;
2-[4-(2-Fluorobenzylthio)-benzylamino]-propanamide;
2-[4-(3-Fluorobenzylthio)-benzylamino]-propanamide;
2-[4-(3-Phenylpropyloxy)-benzylamino]-propanamide;
2-[4-(4-Phenylbutyloxy)-benzylamino]-propanamide;
2-[4-(5-Phenylpentyloxy)-benzylamino]-propanamide;
2-(4-Benzyloxybenzylamino)-3-phenyl-N-methyl-propanamide;
2-(4-Benzyloxybenzylamino)-3-methyl-N-methyl-butanamide;
2-(4-Benzyloxybenzylamino)-2-phenyl-acetamide;
2-[4-(2-Fluorobenzyloxy)-benzylamino]-2-phenyl-acetamide;
2-[4-(3-Fluorobcnzyloxy)-benzylamino]-2-phenyl-acetamide;
2-[4-(2-Fluorobenzyloxy)-benzyl-N-methylamino]-2-phenyl-acetamide;
2-[4-(3-Fluorobenzyloxy)-benzyl-N-methylalmino]-2-phcnyl-acetamide;
2-[4-(3-Chlorobenzyloxy)-benzylamino]-2-phenyl-acetamide;
2-[4-(2-Fluorobenzyloxy)-benzylamino]-2-(2-fluorophenyl)-acetamide;
2-[4-(2-Fluorobenzyloxy)-benzylamino]-2-(3-fluorophenyl)-acetanaide;
2-[4-(3-Fluorobenzyloxy)-benzylamino]-2-(2-fluo.rophenyl)-acetamide;
2-[4-(3-Fluorobencyloxy)-benzylamino]-2-(3-fluorophenyl)-acetamide;
2-[4-(3-Chlorobenzyloxy)-benzylamino]-2-(3-fluorophenyl)-acetamide;
2-(4-(2-Thienyloxy)-benzylamino)-propanamide;
or isomers, mixtures, and pharmaceutically acceptable salts thereof.

6. The use of any one of claims 1-5, wherein the α-aminoamide is (S)-(+)-2-[4-(2- fluorobenzyloxy)-benzylamino]-propanamide.

## Patentansprüche

1. Verwendung einer α-Aminoamid-Verbindung der Formel (1) 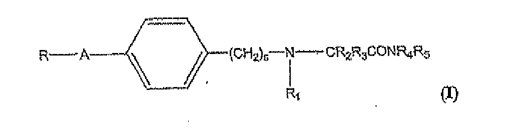 wobei
• A eine -(CH₂)ₙ-X-Gruppe ist, wobei n eine ganze Zahl von 0 bis 5 ist, X CH₂, -O-, -S- oder -NH- ist;
• s 1 oder 2 ist;
• R ein Furyl-, Thienyl- oder Pyridylring oder ein Phenylring ist, gegebenenfalls substituiert durch einen oder zwei Substituenten, die unabhängig voneinander ausgewählt werden aus Halogen, Hydroxy, Cyano, (C₁-C₆)-Alkyl, (C₁-C₆)-Alkoxy oder Trifluoromethyl;
• R₁ Wasserstoff oder (C₁-C₆)-Alkyl oder (C₃-C₇)-Cycloalkyl ist;
• R₂ und R₃ unabhängig ausgewählt werden aus Wasserstoff; (C₁-C₄)-Alkyl, gegebenenfalls substituiert durch Hydroxy oder Phenyl; Phenyl, gegebenenfalls substituiert durch einen oder zwei Substituenten, die unabhängig voneinander ausgewählt werden aus (C₁-C₆)-Alkyl, Halogen, Hydroxy, (C₁-C₆)-Alkoxy oder Trifluoromethyl; oder R₂ und R₃ gemeinsam mit dem Kohlenstoffatom, an das sie gebunden sind, einen (C₃-C₆)-Alkylring bilden; und
• R₄, R₅ unabhängig voneinander Wasserstoff, (C₁-C₆)-Alkyl oder (C₃-C₇)-Cycloalkyl sind; oder R₄ und R₅ gemeinsam mit dem Stickstoffatom, an das sie gebunden sind, einen gesättigten 5 bis 7-atomigen heterocyclischen Ring bilden;
oder Isomere, Gemische und pharmazeutisch verträgliche Salze oder Ester davon, als antientzündliches Mittel zur Herstellung eines Medikamentes für die Behandlung von entzündlichen Erkrankungen.

2. Verwendung nach Anspruch 1, wobei A ausgewählt wird aus -CH₂-, -CH₂-CH₂-, -CH₂-S-, -CH₂-CH₂-S- oder -(CH₂)ₙ-O-; n eine ganze Zahl von 0 bis 5 ist; s 1 oder 2 ist; R ein Phenylring ist, gegebenenfalls substituiert durch einen oder zwei Substituenten, die unabhängig voneinander ausgewählt werden aus Halogen, Trifluoromethyl, Methoxy oder einem Thienylring; R₁ Wasserstoff oder (C₁-C₄)-Alkyl ist; einer von R₂ und R₃ Wasserstoff ist und der andere (C₁-C₄)-Alkyl ist, gegebenenfalls substituiert durch Hydroxy oder Phenyl oder Phenyl, gegebenenfalls substituiert durch ein oder zwei Halogenatome, oder R₂ und R₃ beide Methyl sind oder zusammen mit dem Atom, an das sie gebunden sind, einen Cyclopropyl- oder einen Cyclopentylring bilden; und R₄, R₅ Wasserstoff oder (C₁-C₄)-Alkyl sind oder gemeinsam mit dem Stickstoff, an den sie gebunden sind, einen Pyrrolidin- oder Piperidinring bilden.

3. Verwendung nach Anspruch 1, wobei die Verbindungen in einer Dosis im Bereich von etwa 0,3 bis etwas 100 mg/kg Körpergewicht pro Tag einem Säugetier verabreicht werden.

4. Verwendung nach Anspruch 1, wobei die entzündlichen Erkrankungen ausgewählt werden aus der aus Spondylitis ankylosans; Zervikalarthritis; Fibromyalgie; Gicht, juveniler rheumatoider Arthritis; lumbosakraler A rthritis; Osteoarthritis; Osteoporose; psoriatischer Arthritis; rheumatischer Erkrankung; rheumatoider Arthritis; Ekzem; Psoriasis; Dermatitis; Sonnenbrand; entzündlichen Augenzuständen; Uveitis; Konjunktivitis; entzündlichen Lungenerkrankungen; Asthma; Bronchitis; Ulkus; Gingivitis; Morbus Crohn; atrophischer Gastritis; Gastritis varialoforme; ulzerativer Kolitis; Zöliakie; regionaler Iletis; peptischer Ulzeration; Pyresis; Entzündung des Magen-Darm-Trakts aufgrund von Helicobacter pylori; viszeraler Entzündung; Blasenreizung; Zystitis; entzündlichen neurologischen Erkrankungen des zentralen oder peripheren Nervensystems; multipler Sklerose; entzündlicher Neuropathie; neurologischen Komplikationen von AIDS und weiteren mit Entzündungen verbundenen Erkrankungen oder Störungen bestehenden Gruppe.

5. Verwendung nach einem der Ansprüche 1 bis 4, wobei die Verbindung ausgewählt wird aus 2-(4-Benzyloxybenzylamino)-propanamid; 2-[4-(2-Methoxybenzyloxy)benzylamino]-propanamid; 2-[4-(2-Fluorbenzyloxy)-benzylamino]-propanamid; (S)-(+)-2-[4-(2-Fluorbenzyl-oxy)-benzylamino]-propanamid; 2-[4-(2-Fluorbenzyloxy)-benzylamino]-2-methyl-propanamid; (S)-(+)-2-j4-(3-Fluorbenzyloxy)-benzylamino]-propan-amid-methansulfonat 2-[4-(2-Fluorbenzyloxy)-benzylamino]-N-methyl-propanamid; N-{2-[4-(2-Fluorbenzyloxy)-benzylamino]}-propionyl-pyrrolidin; 2-[4-(3-Methoxybenzyioxy)-benzylamino]-propanamid; 2-[4-(3-Cyano-benzyloxy)-benzylamino]-propanamid; 2-[4-(3-Fluorbenzyloxy)-benzylamino]-propanamid; 2-[4-(3-Fluorbenzyloxy)-benzylamino]-2-methyl-propan-amid; 2-[4-(3-Fluorbenzyloxy)-benzylamino]-N-methyl-propan-amid; N-{2-(4-(3-Fluorbenzyloxy)-benzylamino]}propionyl-pyrrolidin; 2-[4-(4-Fluorbenzyloxy)-benzylamino]-propanamid; 2-[4-(3-Fluorbenzyloxy)-benzylamino]-2-methyl-propanamid; 2-[4-(2-Chlorbenzyloxy)-benzylamino]-propanamid; 2-[4-(3-Chlorbenzyloxy)-benzylamino]-propanamid; 2-(4-Benzyloxybenzylomino)-3-hydroxy-propanamid; 2-[4-(2-Fluorbenzyl-oxy)-benzylamino]-3-hydroxy-propanamid; 2-[4-(3-Fluorbenzyloxy)-benzylamino]-3-hydroxy-propanamid; 2-(4-Benzyloxybenzylamino)-3-hydroxy-N-methyl-propanamid; 2-[4-(2-Fluorbenzyloxy)-benzylamino]-3-hydroxy-N-methyl-propanamid; 2-[4-(3-Fluorbenzyloxy)-benzylamino]-3-hydroxy-N-methyl-propanamid; 2-[4-(2-Chlorbenzyloxy)-benzylannino]-3-hydroxy-N-methyi-propanamid; 2-[4-(3-Cyanabenzyloxy)-benzylamino]-3-hydroxy-N-methyl-propanamid; 2-[4-(3-Cyanobenzyloxy)-benzylamino]-2-methyl-3-hydroxy-N-methyl-propanamid; 2-[4-(3-Chlorbenzyloxy)-phenylethyl-amino]-propanamid; 2-{4-[2-(3-Fluorphenyl)-ethyloxy]benzylamino}-propanamid; 2-{4-[2-(3-Fluorphenyl)-ethyl]-benzylamino}-propanamid; 2-[N-(4-Benzyloxybenzyl)-N-methylamino]-propanamid; 2-{4-[(3-Chlorbenzyl-oxy)-phenylethyl]-amino}-propanamid; 2-[4-Benzylthlobenzylamino]-propanamid; 2-[4-(2-Fluorbenzylthio)-benzyiamino]-propanamid; 2-[4-(3-Fluorbenzylthio)-benzylamino]-prapanamid; 2-[4-(3-Phenylpropyloxy)-benzyiamino]-propanamid; 2-[4-(4-Phenylbutyloxy)-benzylamino]-propanamid; 2-[4-(5-Phenylpentyloxy)-benzylamino]-propanamid; 2-(4-Benzyloxybenzylomino)-3-phenyl-N-methyl-propanamid; 2-(4-Benzyloxybenzylamino)-3-methyl-N-methyl-butanamid; 2-(4-Benzyloxybenzylamino)-2-phenyl-acetamid; 2-[4-(2-Fluorbenzyloxy)-benzylamino]-2-phenyl-acetamid; 2-[4-(3-Fluorbenzyloxy)-benzylamino]-2-phenyl-acetamid; 2-[4-(2-Fluorbenzyloxy)-benzyl-N-methylamino]-2-phenyl-acetamid; 2-[4-(3-Fluorbenzy]oxy)-benzyl-N-methylamino]-2-phenyl-acetamid; 2-[4-(3-Chlorbenzyloxy)-benzylamino]-2-phenyl-acetamid; 2-[4-(2-Fluorbenzyloxy)-benzylamino]-2-(2-fluorphenyl)-acetamid; 2-[4-(2-Fluorbenzyloxy)-benzylamino]-2-(3-fluorphenyl)-acetamid; 2-[4-(3-Fluorbenzyloxy)-benzylamino]-2-(2-fluorphenyl)-acetamid; 2-[4-(3-Fluorbenzyloxy)-benzylamino]-2-(3-fluorphenyl)-acetamid; 2-[4-(3-Chlorbenzyloxy)-benzylamino]-2-(3-fluorphenyl)-acetamid; 2-(4-(2-Thienyloxy)-benzylamino)-propanamid; oder Isomeren, Gemischen und pharmazeutische verträglichen Salzen davon.

6. Verwendung nach einem der Ansprüche 1 bis 5, wobei das α-Aminoamid (S)-(+)-2-[4-(2-Fluorbenzyloxy)-benzylamino]-propanamid ist.

## Revendications

1. Utilisation d'un composé d'α-aminoamide répondant à la formule (I), 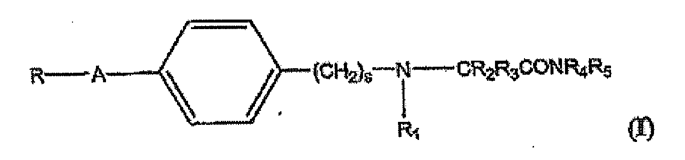 dans laquelle
■ A représente un groupe -(CH₂)ₙ-X- où n représente un entier de 0 à 5, X représente un groupe CH₂, un atome d'oxygène, un atome de soufre ou un groupe -NH- ;
■ s est égal à 1 ou 2 ;
■ R représente un noyau furyle, un noyau thiényle ou un noyau pyridyle, ou représente un noyau phényle substitué de manière facultative par un ou deux substituants choisis de manière indépendante parmi un atome d'halogène, un groupe hydroxyle, un groupe cyano, un groupe alkyle en C₁-C₆, un groupe alcoxy en C₁-C₆ ou un groupe trifluorométhyle ;
■ R₁ représente un atome d'hydrogène ou un groupe alkyle en C₁-C₆ ou un groupe cycloalkyle en C₃-C₇;
■ R₂ et R₃ sont choisis, de manière indépendante, parmi un atome d'hydrogène, un groupe alkyle en C₁-C₄ substitué de manière facultative par un groupe hydroxyle ou par un groupe phényle, un groupe phényle substitué de manière facultative par un ou deux substituants choisis de manière indépendante parmi un groupe alkyle en C₁-C₆, un atome d'halogène, un groupe hydroxyle, un groupe alcoxy en C₁-C₆ ou un groupe trifluorométhyle ; ou bien R₂ et R₃, pris ensemble avec l'atome de carbone auquel ils sont liés, forment un noyau cycloalkyle en C₃-C₆ et
■ R₄, R₅ représentent, de manière indépendante, un atome d'hydrogène, un groupe alkyle en C₁-C₆ ou un groupe cycloalkyle en C₃-C₇; ou bien R₄ et R₅, pris ensemble avec l'atome d'azote auquel ils sont liés, forment un noyau hétérocyclique saturé contenant de 5 à 7 atomes ;
ou d'un de ses isomères, d'un de ses mélanges et de ses esters ou d'un de ses sels pharmaceutiquement acceptables, à titre d'agent anti-inflammatoire pour la préparation d'un médicament destiné au traitement de troubles inflammatoires.

2. Utilisation selon la revendication 1, dans laquelle A est choisi parmi un groupe -CH₂-, un groupe -CH₂-CH₂-, un groupe -CH₂-S-, un groupe -CH₂-CH₂-S- ou un groupe -(CH₂)ₙ-O- ; n représente un entier de 0 à 5 ; s est égal à 1 ou 2 ; R représente un noyau phényle, substitués de manière facultative par un ou deux substituants choisis de manière indépendante parmi un atome d'halogène et un groupe trifluorométhyle, un groupe méthoxy ou un noyau thiényle; R₁ représente un atome d'hydrogène ou un groupe alkyle en C₁-C₄; un des radicaux R₂ et R₃ représente un atome d'hydrogène et l'autre représente un groupe alkyle en C₁-C₄, substitué de manière facultative par un groupe hydroxyle ou par groupe phényle, ou représente un groupe phényle substitué de manière facultative par un ou deux atomes d'halogène, ou bien R₂ et R₃ représentent tous deux un groupe méthyle ou bien peuvent former ensemble, avec l'atome auquel ils sont liés, un noyau cyclopropyle ou un noyau cyclopentyle; et R₄ et R₅ représentent un atome d'hydrogène ou un groupe alkyle en C₁-C₄, ou bien forment, ensemble avec l'atome d'azote auquel ils sont liés, un noyau pyrrolidine ou un noyau pipéridine.

3. Utilisation selon la revendication 1, dans lequel ledit composé est administré à une dose qui se situe dans la plage d'environ 0,3 à environ 100 mg/kg de poids du corps par jour à un mammifère.

4. Utilisation selon la revendication 1, dans laquelle lesdits troubles inflammatoires sont choisis parmi le groupe constitué par la spondylite ankylosante l'arthrite cervicale ; la fibromyalgie ; un trouble intestinal ; l'arthrite rhumatoïde juvénile; l'arthrite lombo-sacrée; l'astéoarthrite; l'ostéoporose; l'arthrite psoriatique; le rhumatisme; l'arthrite rhumatoïde; l'eczéma; le psoriasis; la dermatite; l'érythème solaire; des affections oculaires inflammatoires; l'uvéite; la conjonctivite ; des troubles pulmonaires inflammatoires; I'asthme; la bronchite ; des ulcères ; la gingivite; la maladie de Crohn; la gastrite atrophique ; la gastrite varioliforme; la colite ulcérante; la maladie coeliaque ; l'iléite régionale ; l'ulcère gastroduodénal; le pyrosis; une inflammation du tractus gastro-intestinal due à Helicobacter pylori; une inflammation viscérale; une irritation vésiculaire; la cystite; des troubles neurologiques inflammatoires du système nerveux central ou périphérique ; la sclérose en plaques ; des neuropathies inflammatoires ; des complications neurologiques du SIDA; et d'autres maladies ou troubles associés à des inflammations.

5. Utilisation selon l'une quelconque des revendications 1 à 4, dans laquelle le composé est choisi parmi:
le 2-(4-benzyloxybenzylamino)-propanamide;
le 2-[4-(2-méthoxybenzyloxy)-benzylamino]-propanamide;
le 2-[4-(2-fluorobenzyloxy)-benzylamino]-propanamide;
le (S)-(+)-2-[4-(2-fluorobenzyloxy)-benzylamino]-propanamide;
le 2-[4-(2-fluorobenzyloxy)-benzylamino]-2-méthyl-propanamide;
le (S)-(+)-2-[4-(3-fluorobenzyloxy)-benzylamino]propanamide, méthanesulfonate;
le 2-[4-(2-fluorobenzyloxy)-benzylamino]-N-méthylpropanamide;
la N-{2-[4-(2-fluorobenzyloxy)-benzylamino]}-propionyl-pyrrolidine ;
le 2-[4-(3-méthoxybenzyloxy)-benzylamino]-propanamide;
le 2-[4-(3-cyanobenzyloxy)-benzylamino]-propanamide;
le 2-[4-{3-fluorobenzyloxy)-benzylamino]-propanamide;
le 2-[4-(3-fluorobenzyloxy)-benzylamino]-2-méthyl-propanamide;
le 2-[4-(3-fluorobenzyloxy)-benzylamino]-N-méthyl-propanamide;
la N-{2-(4-(3-fluorobenzyloxy)-benzylamino]}-prapionyl-pyrrolidine;
le 2-[4-(4-fluorobenzyloxy)-benzylamino]-propanamide;
le 2-[4-(3-fluorobenzyloxy)-benzylamino]-2-méthyl-propanamide;
le 2-[4-(2-chlorobenzyloxy)-benzylamino]-propanamide;
le 2-[4-(3-chlorobenzyloxy)-benzylamino]-propanamide;
le 2-(4-benzyloxybenzylamino)-3-hydroxy-propanamide;
le 2-[4-(2-fluorobenzyloxy)-benzylamino]-3-hydroxy-propanamide;
le 2-[4-(3-fluorobenzyloxy)-benzylamino]-3-hydroxy-propanamide;
le 2-(4-benzyloxybenzylamino)-3-hydroxy-N-méthyl-propanamide;
le 2-[4-(2-fluorobenzyloxy)-benzylamino]-3-hydroxy-N-méthylpropanamide;
le 2-[4-(3-fluorobenzyloxy)-benzylamino]-3-hydroxy-N-méthylpropanamide;
le 2-(4-(2-chlorobenzyloxy)-benzylamino]-3-hydroxy-N-méthyl-propanamide;
le 2-[4-(3-cyanobenzyloxy)-benzylamino]-3-hydroxy-N-méthylpropanamide;
le 2-[4-(3-cyanobenzyloxy)-benzylamino]-2-méthyl-3-hydroxy-N-méthyl-propanamide ;
le 2-[4-(3-chlorobenzyloxy)-phényléthylamino}-propanamide;
le 2-{4-[2-(3-fluorophényl)-éthyloxy]benzylamino}-propanamide
le 2-{4-[2-(3-fluorophényl)-éthyl]benzylamino}-propanamide;
le 2-[N-(4-benzyloxybenzyl)-N-méthylamino]-propanamide;
le 2-{4-[(3-clorobenzyloxy)-phényléthyl]-amino}-propanamide;
le 2-[4-benzylthiobenzylamino]-propanamide;
le 2-[4-(2-fluorobenzylthio)-benzylamino]-propanamide;
le 2-[4-(3-fluorobenzylthio)-benzylamino]-propanamide;
le 2-[4-(3-phénylpropyloxy)-benzylamino}-propanamide;
le 2-[4-(4-phénylbutyloxy)-benzylamino]-propanamide;
le 2-[4-(5-phénylpentyloxy)-benzylamino]-propanamide;
le 2-(4-benzyloxybenzylamino)-3-phényl-N-méthyl-propanamide;
le 2-(4-benzyloxybenzylamino)-3-méthyl-N-méthyl-butanamide;
le 2-(4-benzyloxybenzylamino)-2-phényl-acétamide;
le 2-[4-(2-fluorobenzyloxy)-benzylamino]-2-phényl-acétamide;
le 2-[4-(3-fluorobenzyloxy)-benzylamino]-2-phényl-acétamide;
le 2-[4-(2-fluorobenzyloxy)-benzyl-N-méthylamino}-2-phényl-acétamide;
le 2-[4-(3-fluorobenzyloxy)-benzyl-N-méthylamino]-2-phényl-acétamide;
le 2-[4-(3-chlorobenzyloxy)-benzylamino]-2-phényl-acétamide;
le 2-[4-(2-fluorobenzyloxy)-benzylamino]-2-(2-fluorophényl)-acétamide;
le 2-[4-(2-fluorobenzyloxy)-benzylamino]-2-(3-fluorophényl)-acétamide;
le 2-[4-(3-fluorobenzyloxy)-benzylamino]-2-(2-fluorophényl)-acétamide;
le 2-[4-(3-fluorobenzyloxy)-benzylamino]-2-(3-fluorophényl)-acétamide;
le 2-[4-(3-chlorobenzyloxy)-benzylamino]-2-(3-fluorophényl)-acétamide;
le 2-(4-(2-thiényloxy)-benzylamino)-propanamide ;
ou de leurs isomères, de leurs mélanges et de leurs sels pharmaceutiquement acceptables.

6. Utilisation selon l'une quelconque des revendications 1 à 5, dans laquelle l'α-aminoamide est le (S)-(+)-2-[4-(2-fluorabenzyloxy)-benzylamino]-propanamide.
